(19) Europäisches Patentamt
European Patent Office
Office européen des brevets

(11) Publication number: 0 083 332
A2

# (12) EUROPEAN PATENT APPLICATION

(21) Application number: 82870078.1

(22) Date of filing: 20.12.82

(51) Int. Cl.$^3$: C 07 C 101/04
C 07 C 99/00, B 01 J 31/24

(30) Priority: 28.12.81 US 334947

(43) Date of publication of application:
06.07.83 Bulletin 83/27

(84) Designated Contracting States:
BE DE FR GB

(71) Applicant: MONSANTO COMPANY
Patent Department 800 North Lindbergh Boulevard
St. Louis, Missouri 63166(US)

(72) Inventor: Bachman, Gerald Lee
1627 Dunmorr
Des Peres Missouri 63131(US)

(72) Inventor: Sabacky, Milton Jerome
324 Holloway Road
Ballwin Missouri 63011(US)

(74) Representative: Lunt, John Cooper et al,
Monsanto Europe S.A. Patent Department Avenue de Tervuren 270-272 Letter Box No 1
B-1150 Brussels(BE)

## (54) Asymmetric reduction of nitro-containing prochiral compounds.

(57) There is described the heretofore unknown catalytic asymmetric reduction of the nitro group of nonolefinic prochiral substrates, together with a new route to lysine from caprolactam via N-formyl-3-nitro-caprolactam, 3-nitro-caprolactam and 3-aminocaprolactam as intermediates.

EP 0 083 332 A2

# ASYMMETRIC REDUCTION OF NITRO-CONTAINING PROCHIRAL COMPOUNDS

Lysine is a naturally occurring amino acid having significant commercial interest. It is considered nutritionally critical in the diet of man and animals.

In the past, lysine has been synthesized from malonic ester and γ-chlorobutyronitrile (Fischer, Weigert, Ber. 35, 3772 (1902)) and from benzoylpiperidine (von Braun, Ber. 42, 839 (1909)). Past routes for preparing lysine, however, have resulted in a product which is a racemic mixture of the D- and L-enantiomorphs. Since only the L-enantiomorph is nutritionally effective, past methods of preparation have involved expensive and time-consuming processes for resolving the racemic mixture to obtain the L-form.

It has now been found that L-lysine may be prepared via a new route utilizing catalytic asymmetric hydrogenation whereby L-lysine is obtained without the need for expensive and time-consuming resolution procedures. The process includes the heretofore unknown catalytic asymmetric reduction of the nitro group of a nonolefinic prochiral substrate.

Homogeneous catalysts, i.e. those catalysts that are soluble in the reaction mass, have been found to be particularly useful in processes where an asymmetric result is obtained. For instance, it has been

found that when a material, which is capable of forming a racemic mixture, is hydrogenated in the presence of an optically active homogeneous catalyst, one or the other of the possible desired optical enantiomorphs is obtained in the major amount (i.e. in enantiomeric excess) while the other optical enantiomorph is obtained in minor amount.

In the past, catalytic asymmetric hydrogenation has principally concerned the hydrogenation of olefins, e.g. U.S.P. 3,849,480 of common assignment. In accordance with the present invention, however, it has been found that catalytic asymmetric hydrogenation may be applied to prochiral compounds having a nitro group to afford reduction of the nitro group to an amino group. That discovery forms the basis of the present invention.

Accordingly, it is an object of the present invention to provide a novel method for producing L-lysine.

It is a further object of this invention to provide a process for the preparation of optically active compounds containing an amino group by catalytic asymmetric hydrogenation of prochiral substrates containing a nitro group.

## Background of the Invention

As set forth above, there are various methods known for the preparation of D,L-lysine, but such methods must include a resolution process to separate the nutritionally effective L-enantiomorph from the nutritionally ineffective D-enantiomorph.

Further, the homogeneous catalytic reduction of nitro compounds in the synthesis of aliphatic amines is known (Knifton, J. Org. Chem., Volume 40, No. 4, 1975) but that process does not employ asymmetric catalysis and the products prepared therefrom are, accordingly, not optically active.

Still further, the catalytic hydrogenation of nitroesters to amino esters and subsequent hydrolysis to a corresponding amino acid has been shown (Kaji et al, Bull. Chem. Soc. Japan, Vol. 46, 337-338). Such hydrogenation, however, did not involve asymmetric catalysts and only racemic mixtures were obtained.

Additionally, James et al (J.C.S. Chem. Comm., page 574, 1975) discloses the use of a ruthenium chiral phosphine complex, i.e. $[Ru_2X_4\{(+)\text{-}(DIOP)\}_3]$ where X represents Cl or Br and diop is 2,2-dimethyl-1,3-dioxolan-4,5-bis(methylene)bis(diphenylphosphine), in catalytic asymmetric hydrogenation of olefinic substrates in toluene containing triethylamine.

## Summary of the Invention

In accordance with the objects set forth above, the present invention provides a novel method for the preparation of L-lysine via the catalytic asymmetric reduction of 3-nitrocaprolactam to afford 3-aminocaprolactam and subsequent hydrolysis to L-lysine. The process of the invention is accomplished by the use of a catalyst which is a complex of ruthenium containing at least one optically active phosphine or arsine ligand. The ligand may be mono-, bi- or polydentate.

## Description of the Preferred Embodiments

The present invention provides a novel route to L-lysine wherein caprolactam (I) is treated to afford N-formyl-3-nitrocaprolactam (II) which is further treated to afford 3-nitrocaprolactam (III) which is subsequently catalytically asymmetrically reduced in accordance with this invention to afford 3-aminocaprolactam (IV) which is hydrolyzed to afford L-lysine (V) in accordance with the following reaction scheme wherein * denotes an optically active center:

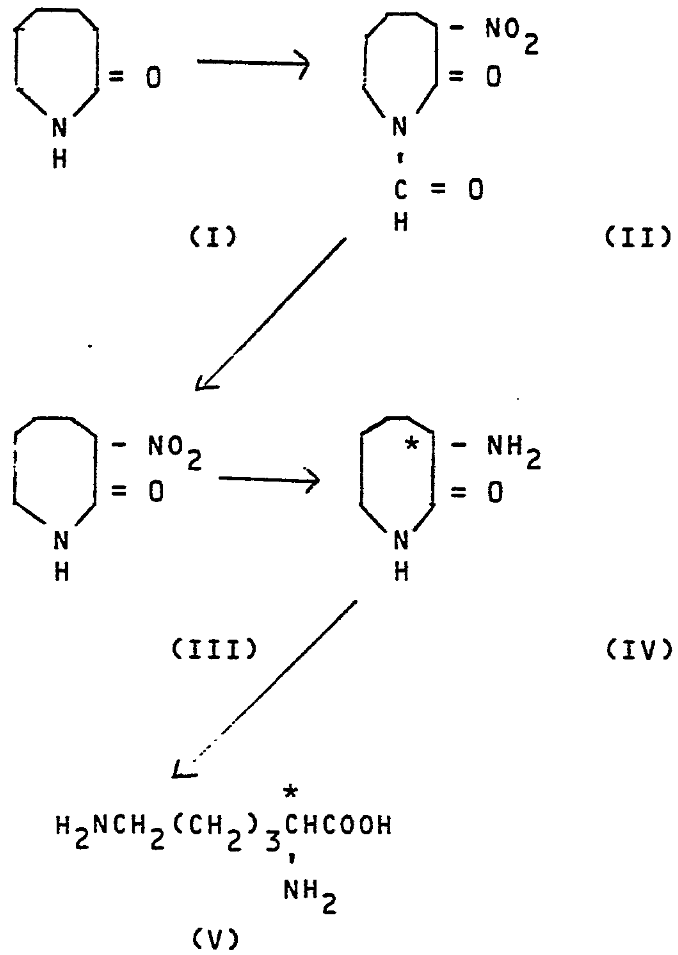

(I) (II) (III) (IV) (V)

In more detail, N-formyl-3-nitrocaprolactam is prepared from caprolactam in accordance with U.S.P. 3,557,093. Thus, phosphorus pentachloride is added to a stirred solution of dimethyl formamide in benzene. To that mixture there is added a solution of caprolactam in benzene and the mixture is stirred. The reaction mixture is slowly added to a solution of sulfuric acid and nitric acid and the reaction mixture is filtered to afford N-formyl-3-nitrocaprolactam. The N-formyl-3-nitrocaprolactam is refluxed in water and cooled to room temperature to afford 3-nitrocaprolactam.

In accordance with the present invention, the 3-nitrocaprolactam is charged to a pressure bomb containing a catalyst which is a complex of ruthenium containing at least one optically active phosphine or arsine ligand and a tertiary amine in a mixture of toluene/ethanol. The bomb is pressured with hydrogen and heated for a sufficient period of time for the reaction to be completed. The solvent is stripped from the reaction mixture, the residue is dissolved in ethanol and treated with anhydrous HCl and the reaction mixture is washed, crystallized and filtered to afford 3-aminocaprolactam hydrochloride.

The 3-aminocaprolactam hydrochloride is dissolved in water and treated with a dilute solution of hydrochloric acid. The mixture is refluxed and the water and excess hydrogen chloride is removed by evaporation leaving a residue which is crystalline L-lysine dihydrochloride.

The solvents which are used in the asymmetric hydrogenation of 3-nitrocaprolactam to afford 3-aminocaprolactam may be any of those solvents in which the lactam or other substrate and the catalyst are soluble and which doesn't poison the catalyst. Such solvents include mixtures of alcohol and aromatic hydrocarbons such as ethanol/toluene and ethanol/benzene, amide solvents such as dimethylformamide and dimethylacetamide, acids and anhydrides such as acetic acid and acetic anhydride and alcohols such as methanol, ethanol and isopropanol.

Ordinarily, the reaction is conducted under a hydrogen pressure of from about 7 to about 350 $kg/cm^2$. Although a lower hydrogen pressure may be utilized, less than 7 $kg/cm^2$ affords a sluggish reaction.

The temperature of the reaction may be from about 50 to about 100°C. Above about 100°C. the reaction components and product may decompose.

It is essential that a base, such as a tertiary amine, e.g. triethylamine, be added to the reaction since

it has been found that tertiary amines activate the nitro group and allow reduction via catalytic asymmetric hydrogenation.

The catalysts which are used in accordance with the present invention comprise a complex of ruthenium containing at least one optically active phosphine or arsine ligand, which ligand may be mono-, bi- or polydentate. The optical activity may reside on the phosphorus atom itself or be present in a substituent attached to the phosphorus atom. A noninclusive listing of types of ruthenium complexes which may be utilized in the process of the invention are set forth below:

1. $RuX_2L_3$
2. $RuX_2L_2L'$
3. $RuX_2(L-L)(L)$
4. $Ru_2X_4(L-L)_3$
5. $Ru_2X_4(L-L)(L-L)'_2$
6. $Ru_2X_4LL'(L-L)_2$
7. $HRuX(L-L)_2$
8. $RuX_2(L-L)_2$

In the above, H represents hydrogen, X represents chlorine, bromine, iodine, alkylcarbonyloxy (e.g. OC(=O)R wherein R represents an alkyl group of 1-8 carbon atoms), 2, 3 and 4 represent integers, L represents an optically active monophosphine or monoarsine ligand, L-L represents an optically active bisphosphine or bisarsine ligand, L' and (L-L)' represent mono- and bisphosphine or arsine ligands which are different than L or L-L.

Exemplary mono-, bi- or polydentate phosphorus-containing ligands which may be utilized in the catalysts which are used in the present invention include the phosphine and arsine ligands described in U.S.P. 3,849,480 (Col. 3, lines 13-44) and U.S.P. 4,008,281 (Col. 2 and Col. 3, lines 1-42), of common assignment, which descriptions are herein specifically incorporated by reference.

A particularly preferred catalyst found useful

in the process of the invention is that of type 3, listed above, where X represents chlorine and L-L represents 2,2-dimethyl-1,3-dioxolan-4,5-bis(methylene)bis(diphenylphosphine) described by James et al, above. Other preferred ligands include 1,2-bis(o-anisylphenylphosphino)-ethane and 1,2-bis(o-anisylcyclohexylphosphino)ethane, phenyl-o-anisylmethylphosphine and methylcyclohexyl-o-anisylphosphine together with the arsine counterparts. Thus, it has been found that optically active forms of the above-described coordination complex catalysts possess the ability to accomplish catalytic asymmetric hydrogenation of prochiral nitro-containing substrates which form an asymmetric carbon atom upon hydrogenation. For example, it is contemplated that, in addition to the reduction of 3-nitrocaprolactam, other prochiral compounds containing a nitro group, as discussed hereinafter, may be catalytically asymmetrically hydrogenated in accordance with the present invention to afford a desired amine-containing enantiomorph.

Thus, nitro-containing prochiral substrates which may be catalytically asymmetrically hydrogenated are represented by those having the formulae:

1) 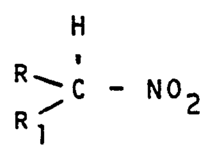 and 2) 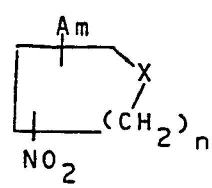

In formula 1, R and $R_1$ are different and each, independently of the other, represents alkyl of 1-8 carbon atoms, e.g. methyl, ethyl, propyl, butyl, amyl, hexyl, heptyl, octyl; carboxyl; phenyl; benzyl; cycloalkyl of 5-7 carbon atoms, e.g. cyclopentyl, cyclohexyl, cycloheptyl; and heterocyclic rings, containing one or more hetero atoms, of 5-7 members containing O, S or N and combinations thereof. Additionally, R and $R_1$ may be substituted to any degree and by any group which does not

interfere with the hydrogenation reaction. Examples of groups which may be substituents on R and $R_1$ include, but are not limited to, alkyl of 1-4 carbon atoms, hydroxyl, carboxyl and derivatives thereof such as the carboxamide and carboxylic ester; oxo (i.e. = O); ethers; phenyl and benzyl.

Examples of prochiral substrates encompassed by formula 1 include α-nitro-β-phenylpropionic acid, 2-nitro-3-(3,4-dihydroxyphenyl) propanoic acid and nitrosuccinic acid which are the prochiral substrate precursors of, respectively, phenylalanine, Dopa and aspartic acid.

In formula 2, X represents $CH_2$, O, S and N, n represents the integers 1, 2 and 3, A represents a group substituted on one or more carbon or nitrogen atoms of the ring and which may be the same as the substituents defined above with relation to R and $R_1$ in formula 1, and m represents an integer which can equal up to the total number of carbon atoms in the ring minus one.

With regard to the 5, 6 and 7-membered ring systems encompassed by formula 2:

1) They may be cycloalkyl or heterocyclic.

2) The nitro group is attached to any ring carbon atom so long as its point of attachment does not introduce an element of symmetry into the molecule, i.e. the molecular configuration must remain asymmetrical.

3) In the cases wherein the compound represented by formula 2 is a cycloalkyl ring, i.e. X = $CH_2$, n = 1, 2 or 3, there must be a substituent on the ring in addition to the nitro group in order that there is no element of symmetry introduced into the molecule. Further, in the case of X = $CH_2$, n = 2, in order to avoid symmetry, the molecule must contain at least one substituent in addition to the nitro group as set forth above, and, in the case where there is only one substituent,

that substituent may be attached at any position except para to the nitro group.

4) Substituents on the ring (i.e. A) may be attached at any position so long as a point of attachment does not introduce an element of symmetry into the molecule. The number of substituents on the ring is of no consequence so long as there is no interference with the hydrogenation reaction.

The concept of symmetry is a complex subject and the entire scope thereof cannot be discussed herein. For a more complete discussion of symmetry, see Advanced Organic Chemistry, 3rd. Ed., G. W. Wheland, pp. 211 et seq (1960).

For purposes of the present invention, a molecule is considered symmetrical when an imaginary plane through the molecule divides the molecule into mirror-image halves. Conversely, an asymmetrical molecule contains no such plane.

Examples of compounds falling within the scope of formula 2 include, but are not limited to, 2-methyl-nitrocyclopentane, 2-hydroxyl-nitrocyclohexane, 3-carboxamido-nitrocycloheptane, 2- or 3-nitrotetrahydrofuran, 2- or 3-nitrotetrahydropyran, 2,3 or 4-nitrohexahydrooxepine, 2- or 3-nitrotetrahydrothiophene, 2- or 3-nitrotetrahydrothiopyran, 2,3 or 4-nitrohexahydroazepine, 2- or 3-nitropyrrolidine, 2- or 3-nitropiperidine, and 2,3 or 4-nitrohexahydrothiepin, including the above compounds containing additional substituents on the ring carbon atoms or nitrogen as set forth above, e.g. 3-nitrocaprolactam.

Additionally, formula 2 represents heterocyclic groups which contain more than one hetero member, e.g. nitromorpholine.

The following examples are illustrative of specific embodiments of the invention. It is understood

that the invention is not limited thereto. In the following examples, enantiomeric excess (i.e. % optical purity) is determined by the following equation (it is understood that the optical activities expressed as the specific rotations are measured in the same solvent).

$$\% \text{ optical purity} = \frac{\text{observed optical activity of the mixture} \times 100}{\text{optical activity of pure enantiomorph}}$$

## Example 1

Preparation of N-formyl-3-nitrocaprolactam and 3-nitrocaprolactam.

In accordance with the procedure of U.S.P. 3,557,093, 105 grams of $PCl_5$ were added to a stirred solution of 19 grams of dimethylformamide in 75 milliliters of benzene. The temperature rose to 35°C. To the mixture was added a solution of 28.3 grams of caprolactam in 100 milliliters of benzene. The mixture was stirred over a period of ten hours and the solvent was stripped on an evaporator. The yellow slurry was added dropwise to a solution of 250 milliliters of 98% sulfuric acid and 50 milliliters of 100% nitric acid over one-half hour at a temperature of 5-8°C. The mixture was stirred for one-half hour after addition in an ice bath. The mixture was then poured onto ice and the solid was filtered and recrystallized from ethanol to afford N-formyl-3-nitrocaprolactam (m.p. 120-122°C.) which was then refluxed in water for 45 min. and recrystallized from water to afford 3-nitrocaprolactam, m.p. 160-162°C.

## Example 2

Preparation of 3-aminocaprolactam.

To a high pressure bomb there was charged 0.20 gram of $Ru_2Cl_4[(-)-(DIOP)]_3$ and a solution of 0.50 gram 3-nitrocaprolactam and 0.68 gram triethylamine in 45 milliliters of a 1:1 solution of toluene/ethanol. The

bomb was evacuated and purged three times with hydrogen and then hydrogen was added to a pressure of 28.1 kg/cm$^2$ gage. Over a period of three days, the pressure was maintained between about 26.4 and 30.9 kg/cm$^2$ gage at a temperature of from 25 to 75°C. At the end of the three-day period the temperature was allowed to return to ambient. The orange reaction mixture was stripped of solvents to yield an appreciable amount of a yellow solid. The yellow solid was triturated with methanol and filtered yielding a purified yellow solid. The solid was dissolved in ten milliliters of ethanol and cooled in an ice bath and dry hydrogen chloride was bubbled into the mixture for ten minutes. Ethanol was stripped and the residue was taken up in ethyl acetate/water. The ethyl acetate layer was drawn off leaving a water layer which was extracted with chloroform. The aqueous solution was filtered and stripped of water. Three milliliters ethanol were added to the solid residue which was allowed to stand at ambient conditions for four hours. All of the material dissolved and a precipitate formed. The precipitate was filtered and washed with ether to yield an off-white solid which was determined to be 3-aminocaprolactam hydrochloride $[\alpha]^{25}_{D}$ + 10.3 in 39% enantiomeric excess.

Example 3

Hydrolysis of 3-aminocaprolactam to afford L-lysine.

In 1.0 gram water there is dissolved 0.1 gram (0.8 mmole) of the 3-aminocaprolactam prepared in Example 2. The resulting mixture is treated with 0.3 gram of 37% HCl. The mixture is stirred at reflux for two hours and the water and excess HCl is removed on a rotary evaporator and the residue is dissolved in 1 ml of hot 95% ethanol. The alcoholic solution is cooled to 25°C. and 1.5 ml of ethyl ether is added slowly with stirring. The crystalline L-lysine dihydrochloride (m.p. 187-188°C.)

is removed by filtration and dried.

In the same general manner as set forth above, α-nitro-β-phenylpropionic acid, 2-nitro-3-(3,4-dihydroxyphenyl) propanoic acid and nitrosuccinic acid afford, respectively, phenylalanine, Dopa and aspartic acid.

While illustrative embodiments of the invention have been described hereinbefore with particularity, it will be understood that various other modifications will be apparent to and can be readily made by those skilled in the art without departing from the spirit and scope of the invention. Accordingly, it is not intended that the scope of the claims appended hereto be limited to the examples and descriptions set forth herein but rather that the claims be construed as encompassing all the features of patentable novelty which reside in the present invention including all features which will be treated as equivalents thereof by those skilled in the art to which the invention pertains.

CLAIMS:

1. Process of asymmetrically hydrogenating a prochiral compound, represented by the formula

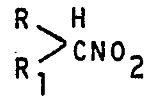

wherein R and $R_1$ are different and each represents alkyl, phenyl, benzyl, cycloalkyl and a heterocyclic ring and wherein R and $R_1$ may be substituted, which comprises hydrogenating said compound in the presence of a base and a catalyst comprising a coordination complex of ruthenium containing at least one optically active ligand selected from the group consisting of phosphine and arsine ligands.

2. Process of Claim 1 wherein said ligand is a phosphine ligand.

3. Process of Claim 2 wherein said ligand is 2,2-dimethyl-1,3-dioxolan-4,5-bis(methylene)bis(diphenylphosphine).

4. Process of Claim 2 wherein said ligand is 1,2-bis(o-anisylphosphino)ethane.

5. Process of Claim 2 wherein said ligand is 1,2-bis(o-anisylcyclohexylphosphino)ethane.

6. Process of Claim 2 wherein said ligand is phenyl-o-anisylmethylphosphine.

7. Process of Claim 2 wherein said ligand is methylcyclohexyl-o-anisylphosphine.

8. Process of Claim 1 wherein said ligand is an arsine ligand.

9. Process of Claim 8 wherein said ligand is 2,2-dimethyl-1,3-dioxolan-4,5-bis(methylene)bis(diphenylarsine).

10. Process of Claim 8 wherein said ligand is 1,2-bis(o-anisylarsino)ethane.

11. Process of Claim 8 wherein said ligand is 1,2-bis(o-anisylcyclohexylarsino)ethane.

12. Process of Claim 8 wherein said ligand is phenyl-o-anisylmethylarsine.

13. Process of Claim 8 wherein said ligand is methylcyclohexyl-o-anisylarsine.

14. Process of Claim 1 wherein said prochiral compound is selected from the group consisting of α-nitro-β-phenyl-propionic acid, 2-nitro-3-(3,4-dihydroxyphenyl)propanoic acid and nitrosuccinic acid.

15. Process of asymmetrically hydrogenating a prochiral compound, represented by the formula

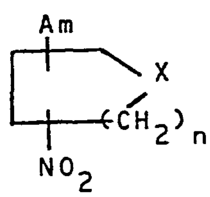

wherein X represents $-CH_2$, >O, >S or -NH, n represents 1, 2 or 3, A represents a substituent and m represents an integer which is equal to the total number of carbons in the ring minus one, which comprises hydrogenating said compound in the presence of a base and a catalyst comprising a coordination complex of ruthenium containing at least one optically active ligand selected from the group consisting of phosphine and arsine ligands.

16. Process of Claim 15 wherein said ligand is a phosphine ligand.

17. Process of Claim 16 wherein said ligand is 2,2-dimethyl-1,3-dioxolan-4,5-bis(methylene)bis(diphenylphosphine).

18. Process of Claim 16 wherein said ligand is 1,2-bis(o-anisylphosphino)ethane.

19. Process of Claim 16 wherein said ligand is 1,2-bis(o-anisylcyclohexylphosphino)ethane.

20. Process of Claim 16 wherein said ligand is phenyl-o-anisylmethylphosphine.

21. Process of Claim 16 wherein said ligand is methylcyclohexyl-o-anisylphosphine.

22. Process of Claim 15 wherein said ligand is an arsine ligand.

23. Process of Claim 22 wherein said ligand is 2,2-dimethyl-1,3-dioxolan-4,5-bis(methylene)bis(diphenylarsine).

24. Process of Claim 22 wherein said ligand is 1,2-bis(o-anisylarsino)ethane.

25. Process of Claim 22 wherein said ligand is 1,2-bis(o-anisylcyclohexylarsino)ethane.

26. Process of Claim 22 wherein said ligand is phenyl-o-anisylmethylarsine.

27. Process of Claim 22 wherein said ligand is methylcyclohexyl-o-anisylarsine.

28. Process of Claim 1 wherein said catalyst comprises a coordination complex selected from the group consisting of

| | |
|---|---|
| $Ru_2X_2L_3$ | $Ru_2X_4(L-L)(L-L)'_2$ |
| $RuX_2L_2L'$ | $Ru_2X_4LL'(L-L)_2$ |
| $RuX_2(L-L)(L)$ | $HRuX(L-L)_2$ |
| $Ru_2X_4(L-L)_3$ | $RuX_2(L-L)_2$ |

wherein X represents chlorine, bromine, iodine or alkylcarbonyloxy wherein the alkyl group contains from 1-8 carbon atoms, L represents an optically active monophosphine ligand, L-L represents an optically active bisphosphine ligand, L' and (L-L)' represent monophosphine and bisphosphine ligands which are different than L or L-L and 2, 3 and 4 represent integers.

29. Process for preparing L-lysine which comprises hydrogenating 3-nitrocaprolactam in the presence of a base and a catalyst comprising a coordination complex of ruthenium containing at least one optically active ligand selected from the group consisting of

phosphine and arsine ligands to afford 3-aminocaprolactam and hydrolyzing said 3-aminocaprolactam to afford L-lysine.

30. Process of Claim 29 wherein said ligand is a phosphine ligand.

31. Process of Claim 29 wherein said ligand is an arsine ligand.

32. Process of Claim 29 wherein said ligand is 2,2-dimethyl-1,3-dioxolan-4,5-bis(methylene)bis(diphenylphosphine).

33. Process of Claim 29 wherein said ligand is 1,2-bis(o-anisylphenylphosphino)ethane.

34. Process of Claim 29 wherein said ligand is 1,2-bis(o-anisylcyclohexylphosphino)ethane.

35. Process of Claim 29 wherein said ligand is phenyl-o-anisylmethylphosphine.

36. Process of Claim 29 wherein said ligand is methylcyclohexyl-o-anisylphosphine.

37. Process for preparing L-lysine which comprises reacting caprolactam with a formylating agent and a nitrating agent to afford N-formyl-3-nitrocaprolactam, removing said formyl group to afford 3-nitrocaprolactam, hydrogenating said 3-nitrocaprolactam in the presence of a base and a catalyst comprising a coordination complex of ruthenium containing at least one optically active phosphine ligand to afford 3-aminocaprolactam and hydrolyzing said 3-aminocaprolactam to afford L-lysine.

38. Process of Claim 37 wherein said ligand is 2,2-dimethyl-1,3-dioxolan-4,5-bis(methylene)bis(diphenylphosphine).

39. Process of Claim 37 wherein said ligand is 1,2-bis(o-anisylphenylphosphino)ethane.

40. Process of Claim 37 wherein said ligand is 1,2-bis(o-anisylcyclohexylphosphino)ethane.

41. Process of Claim 37 wherein said ligand is phenyl-o-anisylmethylphosphine.

42. Process of Claim 37 wherein said ligand is methylcyclohexyl-o-anisylphosphine.

43. Process of preparing L-lysine which comprises hydrogenating 3-nitrocaprolactam in the presence of a base and a catalyst comprising a coordination complex selected from the group consisting of:

| | |
|---|---|
| $Ru_2X_2L_3$ | $Ru_2X_4(L\text{-}L)(L\text{-}L)'_2$ |
| $RuX_2L_2L'$ | $Ru_2X_4LL'(L\text{-}L)_2$ |
| $RuX_2(L\text{-}L)(L)$ | $HRuX(L\text{-}L)_2$ |
| $Ru_2X_4(L\text{-}L)_3$ | $RuX_2(L\text{-}L)_2$ |

wherein X represents chlorine, bromine, iodine or alkylcarbonyloxy wherein the alkyl group contains from 1-8 carbon atoms, L represents an optically active monophosphine ligand, L-L represents an optically active bisphosphine ligand, L' and (L-L)' represent monophosphine and bisphosphine ligands which are different than L or L-L and 2, 3 and 4 represent integers.

44. Process of Claim 43 wherein said complex is $RuX_2L_3$.

45. Process of Claim 43 wherein said complex is $RuX_2L_2L'$.

46. Process of Claim 43 wherein said complex is $RuX_2(L\text{-}L)(L)$.

47. Process of Claim 43 wherein said complex is $Ru_2X_4(L\text{-}L)_3$.

48. Process of Claim 43 wherein said complex is $Ru_2X_4(L\text{-}L)(L\text{-}L)'_2$.

49. Process of Claim 43 wherein said complex is $Ru_2X_4LL'(L\text{-}L)_2$.

50. Process of Claim 43 wherein said complex is $HRuX(L\text{-}L)_2$.

51. Process of Claim 43 wherein said complex is $RuX_2(L\text{-}L)_2$.